(19) Europäisches Patentamt
European Patent Office
Office européen des brevets

(11) Publication number: 0 092 073 B1

(12) **EUROPEAN PATENT SPECIFICATION**

(45) Date of publication of patent specification: **30.03.88**

(51) Int. Cl.$^4$: **C 07 J 51/00,** C 07 J 9/00, A 61 K 31/575

(21) Application number: **83103133.1**

(22) Date of filing: **29.03.83**

(54) **Magnesium salt of chenodeoxycholic acid and ursodeoxycholic acid, the process for its preparation, and therapeutic compositions which contain it as active principle.**

(30) Priority: **15.04.82 IT 2075782**

(43) Date of publication of application: **26.10.83 Bulletin 83/43**

(45) Publication of the grant of the patent: **30.03.88 Bulletin 88/13**

(84) Designated Contracting States: **AT BE CH DE FR GB LI LU NL SE**

(56) References cited:
**DE-C- 254 530**
**US-A-2 429 899**

(73) Proprietor: **SCHWARZ ITALIA S.p.A.**
**Via Emilia 99**
**I-20075 S. Grato-Lodi (Milano) (IT)**

(72) Inventor: **Garzia, Aldo**
**Viale Rimembranze 2**
**Lodi (Milano) (IT)**
Inventor: **Bottazzi, Andrea**
**Via Dall'Oro 14**
**Lodi (Milano) (IT)**

(74) Representative: **Aimi, Luciano et al**
**c/o Società Italiana Brevetti S.p.A.**
**Via Carducci 8**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 092 073 B1

**Description**

This invention relates to a new magnesium salt of chenodeoxycholic acid and ursodeoxycholic acid, the process for its preparation, and therapeutic compositions containing it as active principle which are useful for dissolving bile calculi and preventing their formation.

One of the main bile acids which form from the cholesterol in the liver of animals is known to be chenodeoxycholic acid (hereinafter known as CDCA), namely 17β-(1-methyl-3-carboxypropyl)-ethiocholane-3α,7α-diol

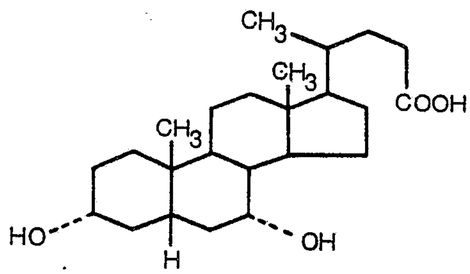

CDCA is prepared in large quantities at the present time from natural sources, for example from ox bile.

The determining physiological action of CDCA is the dissolving of bile lipids, in particular cholesterol, essentially by detergent action. In reality, CDCA is used in human therapy for dissolving bile calculi, but has the drawback of not being able to be used at sufficiently high dosages to give rapid action because of its side effects, of which the main one is the manifestation of diarrhoea, which can also be of a serious extent.

Ursodexoycholic acid (known hereinafter as UDCA), namely 17β-(1-methyl-3-carboxypropyl)-ethiocholane-3α,7β-diol

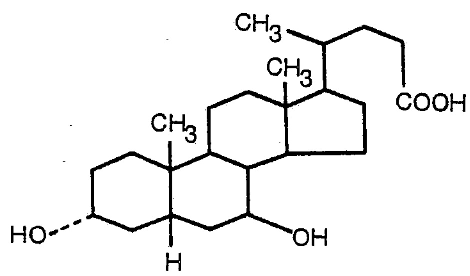

is the epimer of CDCA because of the hydroxyl group in position 7. UDCA is produced at the present time on an individual scale by extraction from ox bile.

It has been found that UDCA performs its biological action more by interfering in the cholesterol biosynthesis than by dissolving its crystals as a detergent.

It is also used in medicine in the treatment of bile calculosis in order to control cholesterol synthesis.

It has however been found that in administering either of the two said bile acids, it is not possible to simultaneously attain optimum conditions for the prophylaxis and treatment of the bile calculi, because CDCA dissolves the already formed cholesterol calculi but does not prevent their formation, whereas UDCA inhibits the formation of calculi, but once formed is unable to dissolve them.

The present invention provides a new compound constituted by the double magnesium salt of chenodeoxycholic acid and ursodeoxycholic acid, in which one atom of magnesium is bonded to one molecule of each of the said acids. This new compound has shown elevated therapeutic efficiency in the treatment and prevention of bile calculi. In practice, it enables the two bile acids to be simultaneously administered associated with each other in a single molecule consisting of said double magnesium salt, i.e. in a stable form of easy and reliable dosage. The association of the two active principles in the form of the double magnesium salt leads unexpectedly to the complete prevention of bile calculi and therapeutic effects which are greater than those which could have been expected from a mixture of the two products.

It should also be noted that tests carried out with the corresponding calcium salt have given results which are completely negative.

The Mg salt has also demonstrated the advantage of acting as a prodrug, in that being practically insoluble in water it is hydrolysed slowly by the gastric juice, to gradually and continuously supply its two component acids for the circulation.

Synthesis of the new compound

According to the present invention, chenodeoxycholic acid is reacted with a magnesium alkoxide at a temperature not exceeding 20°C, in the presence of either the alcohol corresponding to the alkoxide or a

different alcohol. Subsequently, without separating the formed alkoxy-magnesium chenodeoxycholate from the reaction solvent, an equivalent quantity of ursodeoxycholic acid is added to the reaction mixture. The reaction solvent is then evaporated to dryness. All stages of the process take place in an anhydrous environment under a stream of nitrogen.

The Mg alkoxide must be poured slowly into the chenodeoxycholic solution in order to prevent formation of an Mg salt of only chenodeoxycholic acid.

Mg ethoxide is generally used, together with anhydrous ethanol as the reaction medium.

The yield is practically quantitative.

The double magnesium salt has a melting point of 247°—250°C. Its structure was confirmed by IR and NMR spectra. Its empirical formula is $C_{48}H_{78}O_8Mg$.

Example of preparation of the double salt

11.4 g (0.1 moles) of magnesium ethoxide are dissolved under a stream of nitrogen in 400 mg of anhydrous ethyl alcohol.

After dissolution is complete, said solution is added slowly to a solution of 39.25 g (0.1 moles) of chenodeoxycholic acid in 150 ml of anhydrous ethyl alcohol. The procedure is carried out at a temperature not exceeding 20°C.

After a few minutes, a second solution prepared from 39.25 g of ursodeoxycholic acid in 150 ml of anhydrous ethanol is added to the previous solution.

The ethyl alcohol is then evaporated off under reduced pressure at a temperature less than 40°C. A dense residue is obtained which when taken up in acetone produces a solid grey-white product. On drying under vacuum until constant weight, about 80.5 g of the magnesium salt of chenodeoxycholic acid ursodeoxycholic acids are obtained.

The yield is practically quantitative.

Melting point 247—250°C; empirical formula $C_{48}H_{78}O_8Mg$; molecular weight 807.42.

Elementary analysis of the double salt gave the following results:

| for $C_{48}H_{78}O_8Mg$ | Calc. % | Found % |
|---|---|---|
| C | 71.40 | 71.52 |
| H | 9.74 | 9.83 |
| O | 15.85 | 15.72 |
| Mg | 3.01 | 2.93 |

Solubility: insoluble in water, fairly soluble in alcohol.

The compound has confirming IR and NMR spectra.

The magnesium salt of chenodeoxycholic acid melts at about 290°C, and the magnesium salt of ursodeoxycholic acid melts at 257—260°C.

The mixture of the two, in equal parts, melts at 283—290°C.

Pharmacological trials

A summary is given hereinafter of the large number of trials carried out in order to evaluate the new compound of the present invention from the pharmacological aspect. It has been amply shown that the double magnesium salt of chenodeoxycholic acid and ursodeoxycholic acid (known hereinafter as Mg-UCD) is a drug of exceptional value in the prophylaxis and treatment of bile calculosis and in dyspeptic syndromes.

As stated, although CDCA and UDCA are structurally very similar, the pharmacological properties of the two bile acids are qualitatively and quantitatively different.

In particular, chenodeoxycholic acid has greater detergent activity, and is therefore able to dissolve the bile lipids, and in particular cholesterol, to a greater extent. Ursodeoxycholic acid is more effective on the metabolic plane, in that it reduces cholesterol bile secretion to a greater extent.

If only one of the two bile acids is administered, it is not possible to simultaneously attain optimum conditions for dissolving the cholesterol calculi.

By means of controlled study carried out in comparison with the free acids, it has been shown that by administering relatively small doses of the salt Mg-UCD, greater effects can be obtained than with double doses of each of the acids administered separately.

The new salt is more advantageous than the individually administered bile acids from the tolerability aspect. CDCA leads to diarrhoea, and, in certain cases, transaminase increase, while UDCA tends to cause constipation. No significant side effects were noticed in patients treated with the salt.

Finally, the sale possesses a modest anti-dislipidemic effect, in particular in the hypotriglyceridemic sense, which is attained with much smaller doses than those required in treatment with the individual bile acids.

A. Lethality, toxicity and tolerability

Unless otherwise specified, the research was carried out using: a) Mg-UCD capsules as such; b) the powdered contents of Mg-UCD capsules; CDCA (chenodeoxycholic acid) and UDCA (ursodeoxycholic acid)

as pure substance which in all cases was partly dissolved and partly suspended under warm conditions (T = 30°C) in 10% gum arabic mucilage (so as to administer 10—40 ml of liquid per kg orally). In certain cases, namely in the experiments involving parenteral administration, the active principle of Mg-UCD, the chenodeoxycholic acid and ursodeoxycholic acid were dissolved in distilled water of slightly alkaline pH (5—10 ml/kg).

The generally acute lethality, toxicity and tolerability were administered by oral administration of the Mg-UCD (by gastric probe) in the Mus musculus, rat and dog. Animals of both sexes were used, none of which were pregnant. They were fasted for about 12 hours before treatment (but allowing them free access to water). The investigations were carried out using the Mg-UCD in powder form. Comparative investigations were carried out on rats and Mus musculus using the active principle by endoperitoneal administration, and chenodeoxycholic acid and ursodeoxycholic acid by oral administration (using a gastric probe). Each dose was administered to 20 rats or Mus musculus (10 male and 10 female) and to eight dogs (4 male and 4 female). The animals were observed attentively during the 7 days following administration, and any lethality, toxic symptoms, disturbances, functional variations determinable with the eye, behaviour etc. were noted.

The $LD_{50}$ was determined 7 days after administration using the probit method.

The overall research indicates that:

a) both in the case of the rat and Mus musculus, powdered Mg-UCD administered orally (by gastric probe) shows toxic-lethal effects during the 7 days following treatment only in the case of doses per kg which are many times greater than those recommended for human therapy, and gives rise to toxic-lethal effects which are decidedly less than those observed in the case of chenodeoxycholic acid and ursodeoxycholic acid (Tables 1 and 2); a histological examination of the main organs (liver, kidney, spleen, lung, brain) of the deceased animals did not show any specific lesions (turbid swelling and venous stasis) in the case of the liver and kidney; significant differences between the two sexes and two types of animals were not observed.

b) in the case of dogs, Mg-UCD administered orally (by gastric probe) does not show (Tables 1 and 2) during the 7 days following treatment any deaths or any significant toxic symptomatology, even in the case of individual doses per kg corresponding to 2500 mg of active principle (600 Dts/kg) (Tables 1 and 2).

It has also been shown that in the case of rats and Mus musculus, the active principle of Mg-UCD when administered endoperitoneally demonstrates low toxic-lethal effects 7 days from treatment (Table 3).

TABLE 1

Acute lethality and toxicity of the product Mg-UCD, of chenodeoxycholic acid or ursodeoxycholic acid administered orally (by gastric probe) in the Mus musculus, rat and dog. Each dose was administered to 10 rats or Mus musculus and to 4 dogs (M or F)

| Animal species | Treatment mg/kg | % mortality at following times after administration 24 h | 7 days | Toxic symptomatology Latency | Type |
|---|---|---|---|---|---|
| M. musculus M | Mg-UCD (250 mg/kg; 60 Dts/kg) | 0 | 0 | | NN |
| M. musculus M | Mg-UCD (2500 mg/kg; 600 Dts/kg) | 0 | 0 | 5—10 min | D |
| M. musculus M | Mg-UCD (5000 mg/kg; 1200 Dts/kg) | 0 | 0 | 5—10 min | D |
| M. musculus M | Mg-UCD (10000 mg/kg; 2400 Dts/kg) | 10 | 10 | 5—10 min | D |
| M. musculus F | Mg-UCD (250 mg/kg; 60 Dts/kg) | 0 | 0 | | NN |
| M. musculus F | Mg-UCD (2500 mg/kg; 600 Dts/kg) | 0 | 0 | 5—10 min | D |
| M. musculus F | Mg-UCD (5000 mg/kg; 1200 Dts/kg) | 0 | 0 | 5—10 min | D |
| M. musculus F | Mg-UCD (10000 mg/kg; 2400 Dts/kg) | 10 | 10 | 5—10 min | D |
| Rat M | Mg-UCD (250 mg/kg; 60 Dts/kg) | 0 | 0 | | NN |
| Rat M | Mg-UCD (2500 mg/kg; 600 Dts/kg) | 0 | 0 | 5—10 min | D |
| Rat M | Mg-UCD (5000 mg/kg; 1200 Dts/kg) | 0 | 0 | 5—10 min | D |
| Rat M | Mg-UCD (10000 mg/kg; 2400 Dts/kg) | 10 | 10 | 5—10 min | D |
| Rat F | Mg-UCD (250 mg/kg; 60 Dts/kg) | 0 | 0 | | NN |
| Rat F | Mg-UCD (2500 mg/kg; 600 Dts/kg) | 0 | 0 | 5—10 min | D |
| Rat F | Mg-UCD (5000 mg/kg; 1200 Dts/kg) | 0 | 0 | 5—10 min | D |
| Rat F | Mg-UCD (10000 mg/kg; 2400 mg/kg) | 10 | 10 | 5—10 min | D |
| Dog M | Mg-UCD (250 mg/kg; 60 Dts/kg) | 0 | 0 | | NN |
| Dog M | Mg-UCD (2500 mg/kg; 600 Dts/kg) | 0 | 0 | 5—10 min | D |
| Dog F | Mg-UCD (250 mg/kg; 60 Dts/kg) | 0 | 0 | | NN |
| Dog F | Mg-UCD (2500 mg/kg; 600 Dts/kg) | 0 | 0 | 5—10 min | D |
| M. musculus M | Chenodeoxycholic acid 2000 | 0 | 0 | 5—10 min | D |
| M. musculus M | Chenodeoxycholic acid 4000 | 30 | 30 | 5—10 min | D |
| M. musculus M | Chenodeoxycholic acid 8000 | 80 | 80 | 5—10 min | D |
| M. musculus F | Chenodeoxycholic acid 2000 | 0 | 0 | 5—10 min | D |
| M. musculus F | Chenodeoxycholic acid 4000 | 30 | 30 | 5—10 min | D |
| M. musculus F | Chenodeoxycholic acid 8000 | 80 | 80 | 5—10 min | D |

TABLE 1 (continued)

Acute lethality and toxicity of the product Mg-UCD, of chenodeoxycholic acid or ursodeoxycholic acid administered orally (by gastric probe) in the Mus musculus, rat and dog. Each dose was administered to 10 rats or Mus musculus and to 4 dogs (M or F)

| Animal species | Treatment mg/kg | % mortality at following times after administration 24 h | 7 days | Toxic symptomatology Latency | Type |
|---|---|---|---|---|---|
| Rat M | Chenodeoxycholic acid 2000 | 0 | 0 | 5—10 min | D |
| Rat M | Chenodeoxycholic acid 4000 | 40 | 40 | 5—10 min | D |
| Rat M | Chenodeoxycholic acid 8000 | 80 | 80 | 5—10 min | D |
| Rat F | Chenodeoxycholic acid 2000 | 0 | 0 | 5—10 min | D |
| Rat F | Chenodeoxycholic acid 4000 | 30 | 30 | 5—10 min | D |
| Rat F | Chenodeoxycholic acid 8000 | 90 | 90 | 5—10 min | D |
| M. musculus M | Ursodeoxycholic acid 2000 | 0 | 0 | 5—10 min | D |
| M. musculus M | Ursodeoxycholic acid 4000 | 20 | 20 | 5—10 min | D |
| M. musculus M | Ursodeoxycholic acid 8000 | 60 | 60 | 5—10 min | D |
| M. musculus F | Ursodeoxycholic acid 2000 | 0 | 0 | 5—10 min | D |
| M. musculus F | Ursodeoxycholic acid 4000 | 20 | 20 | 5—10 min | D |
| M. musculus F | Ursodeoxycholic acid 8000 | 60 | 70 | 5—10 min | D |
| Rat M | Ursodeoxycholic acid 2000 | 0 | 0 | 5—10 min | D |
| Rat M | Ursodeoxycholic acid 4000 | 10 | 10 | 5—10 min | D |
| Rat M | Ursodeoxycholic acid 8000 | 70 | 70 | 5—10 min | D |
| Rat F | Ursodeoxycholic acid 2000 | 0 | 0 | 5—10 min | D |
| Rat F | Ursodeoxycholic acid 4000 | 10 | 10 | 5—10 min | D |
| Rat F | Ursodeoxycholic acid 8000 | 70 | 60 | 5—10 min | D |

NN = no symptomatology; D = depression

TABLE 2

$LD_{50}$ 7 days after treatment in various animal species

| Animal species | Treatment | $LD_{50}$ (mg/kg with reliability limits) 7 days after treatment | Coefficients of regression line b | a | Linearity $X^2$ |
|---|---|---|---|---|---|
| M. musculus M | Mg-UCD *po* | 13100 (10515—16319) | 6.73 | −22.73 | 0.82 |
| M. musculus F | Mg-UCD *po* | 14011 (10884—18033) | 6.27 | −21.01 | 0.46 |
| Rat M | Mg-UCD *po* | 14011 (10884—18033) | 6.27 | −21.01 | 0.46 |
| Rat F | Mg-UCD *po* | 13100 (10515—16319) | 6.73 | −22.73 | 0.82 |
| Dog M | Mg-UCD *po* | greater than 2500 mg/kg (greater than 600 Dts/kg) | | | |
| Dog F | Mg-UCD *po* | greater than 2500 mg/kg (greater than 600 Dts/kg) | | | |
| M. musculus M | Chenodeoxycholic acid *po* | 5280 (3981—7003) | 4.81 | −12.91 | 0.07 |
| M. musculus F | Chenodeoxycholic acid *po* | 5280 (3981—7003) | 4.81 | −12.91 | 0.07 |
| Rat M | Chenodeoxycholic acid *po* | 4009 (3624—6627) | 4.36 | −11.09 | 0.46 |
| Rat F | Chenodeoxycholic acid *po* | 4886 (3833—6228) | 5.97 | −17.03 | 0.00 |
| M. musculus M | Ursodeoxycholic acid *po* | 5628 (4333—7311) | 5.44 | −15.42 | 0.01 |
| M. musculus F | Ursodeoxycholic acid *po* | 6139 (4518—8338) | 4.62 | −12.53 | 0.00 |
| Rat M | Ursodeoxycholic acid *po* | 6486 (4860—8658) | 5.28 | −15.17 | 0.17 |
| Rat F | Ursodeoxycholic acid *po* | 7333 (5304—10137) | 5.09 | −14.68 | 0.50 |
| M. musculus M | Mg-UCD *ip* | 2455 (1984—3039) | 4.36 | −9.79 | 0.93 |
| M. musculus F | Mg-UCD *ip* | 2455 (1984—3039) | 4.36 | −9.79 | 0.93 |
| Rat M | Mg-UCD *ip* | 2288 (1902—2751) | 5.48 | −13.41 | 0.22 |
| Rat F | Mg-UCD *ip* | 2449 (2063—2906) | 5.97 | −15.25 | 0.00 |

TABLE 3

Lethality and toxicity of the active principle of the product Mg-UCD administered endoperitoneally in the Mus musculus and rat. Each dose was administered to 20 animals (10 males and 10 females)

| Animal species | Treatment mg/kg | % mortality at the following times after administration 24 h | 7 days | Toxic symptomatology Latency | Type |
|---|---|---|---|---|---|
| M. musculus M | 1000 | 0 | 0 | 1—2 min | D |
| M. musculus M | 2000 | 10 | 10 | 1—2 min | D |
| M. musculus M | 4000 | 60 | 60 | 1—2 min | D |
| M. musculus F | 1000 | 0 | 0 | 1—2 min | D |
| M. musculus F | 2000 | 10 | 10 | 1—2 min | D |
| M. musculus F | 4000 | 70 | 70 | 1—2 min | D |
| Rat M | 1000 | 0 | 0 | 1—2 min | D |
| Rat M | 2000 | 10 | 10 | 1—2 min | D |
| Rat M | 4000 | 70 | 70 | 1—2 min | D |
| Rat F | 1000 | 0 | 0 | 1—2 min | D |
| Rat F | 2000 | 10 | 10 | 1—2 min | D |
| Rat F | 4000 | 60 | 60 | 1—2 min | D |

D = depression

B — Action on the formation of cholesterol calculi in the gall-bladder

The investigations were carried out on hampsters (of female sex, but excluding those in a state of pregnancy) having an initial weight of 75—90 g. They were treated for 80 days with oral administration (by gastric probe) of Mg-UCD (10—20 mg/kg/day), chenodeoxycholic acid (10—20 mg/kg/day) or ursodeoxycholic acid (10—20 mg/kg/day) (Tables 4 and 5). The animals were maintained for 80 consecutive days on a Nossan feed diet for rats, enriched with cholesterol (2 mg/g of food).

Each experimental group consisted of 10 units. After 80 days, the animals were narcotised with ethyl ether, and the bile was withdrawn, together with the calculi and crystals from the gall-bladder as described by Pearlman and coll. in Gastroenterology 77, 634 (1979).

The bile acid, phospholipid, cholesterol, cholic acid, chenodeoxycholic acid, ursodeoxycholic acid, DCA (deoxycholic acid) and LCA (lithocholic acid) contents were evaluated in the bile by the methods described by Pearlman and coll. (see above).

The results shows that the product Mg-UCD when administered orally to the hampster hinders the appearance of cholesterol calculi and crystals in the gall-bladder in a dose-treatment manner which is more intense than chenodeoxycholic acid or ursodeoxycholic acid (Table 4). It also reduces the excretion of cholesterol, phospholipids, cholic acid and DCA, while increasing the excretion of chenodeoxycholic acid and ursodeoxycholic acid (Table 5), with a slight increase in LCA. These effects are greater than those observed with chenodeoxycholic acid and ursodeoxycholic acid administered alone.

On the basis of the many pharmacological tests carried out, therapeutic doses for human beings have been established at 300 mg of Mg-UCD, twice a day.

The new salt prepared as described can be administered in gelatin capsules either containing or not containing excipients such as stearates, lactose, starch or mixtures thereof.

Tables containing 300 mg of salt can be prepared with the same excipients.

TABLE 4

| No. Animals | Treatment mg/kg/day | Body weight (g ± s.e.) after following days 0 | 80 | % animals with calculi | crystals |
|---|---|---|---|---|---|
| 10 | Controls (normal) | 82 ± 5 | 148 ± 2 | 0 | 0 |
| 10 | Controls (diet) | 78 ± 4 | 146 ± 7 | 90 | 100 |
| 10 | Mg-UCD (10) | 82 ± 6 | 152 ± 8 | 20 | 20 |
| | Mg-UCD (20) | | | 0 | 0 |
| 10 | Chenodeoxycholic acid (10) | 77 ± 4 | 148 ± 9 | 50 | 30 |
| | Chenodeoxycholic acid (20) | | | 0 | 0 |
| 10 | Ursodeoxycholic acid (10) | 79 ± 5 | 141 ± 7 | 40 | 20 |
| | Ursodeoxycholic acid (20) | | | 0 | 0 |

TABLE 5

Bile lipids (expressed as molar % ± s.e.) Bile acids (expressed as molar % ± s.e.)

| Treatment mg/kg/day | Bile acids | Phospho-lipids | Choles-terol | Cholic acid | Cheno-deoxy-cholic acid | Urso-deoxy-cholic DCA | LCA | |
|---|---|---|---|---|---|---|---|---|
| Normal controls | 80.2 ± 3.9 | 16.8 ± 1.5 | 3.9 ± 0.3 | 48.0 ± 5.2 | 21.8 ± 2.3 | 5.0 ± 0.1 | 27.9 ± 2.7 | 0 |
| Diet controls | 72.8 ± 4.6 | 16.2 ± 1.5 | 12.8 ± 0.5 | 55.3 ± 8.1 | 22.9 ± 2.2 | 4.8 ± 0.2 | 20.6 ± 4.4 | 0 |
| Mg-UCD (10) | 78.6 ± 5.3 | 15.6 ± 1.7 | 6.9 ± 0.4 | 18.1 ± 2.6 | 28.6 ± 2.9 | 21.7 ± 2.9 | 13.4 ± 0.5 | 2.4 ± 0.1 |
| Mg-UCD (20) | 80.7 ± 4.7 | 14.7 ± 1.9 | 4.7 ± 0.3 | 11.4 ± 1.7 | 24.4 ± 3.1 | 54.9 ± 3.6 | 10.5 ± 0.4 | 3.6 ± 0.2 |
| Chenodeoxycholic acid (10) | 79.5 ± 5.6 | 16.0 ± 1.8 | 7.2 ± 0.2 | 29.6 ± 2.6 | 42.1 ± 3.6 | 6.6 ± 0.4 | 12.7 ± 0.6 | 3.2 ± 0.1 |
| Chenodeoxycholic acid (20) | 80.2 ± 3.8 | 15.8 ± 1.3 | 5.9 ± 0.3 | 18.7 ± 2.1 | 66.0 ± 4.7 | 7.8 ± 0.3 | 10.4 ± 0.2 | 3.4 ± 0.3 |
| Ursodeoxycholic acid (10) | 79.7 ± 4.1 | 15.5 ± 1.0 | 7.4 ± 0.4 | 30.2 ± 4.1 | 12.4 ± 2.6 | 36.2 ± 1.4 | 12.9 ± 0.8 | 2.5 ± 0.1 |
| Ursodeoxycholic acid (20) | 81.3 ± 4.3 | 15.8 ± 1.0 | 5.7 ± 0.2 | 17.9 ± 2.1 | 4.5 ± 0.9 | 72.8 ± 4.7 | 10.5 ± 0.6 | 3.9 ± 0.2 |

**Claims**

1. A double magnesium salt of chenodeoxycholic acid and ursodeoxycholic acid, of formula

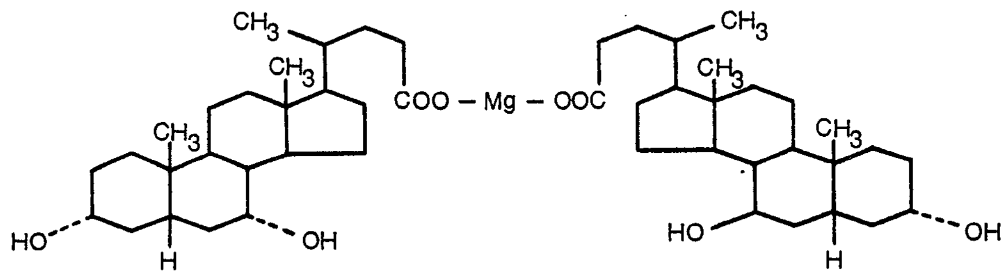

2. A process for preparing the compound as claimed in claim 1, characterised by reacting a magnesium alkoxide with chenodeoxycholic acid in an anhydrous alcoholic solution at a temperatuire not exceeding 20°C, to form the alkoxy-magnesium chenodeoxycholate, then reacting this latter in the same alcoholic solution with ursodeoxychloric acid.

3. A process as claimed in claim 2 using magnesium ethoxide and ethyl alcohol as the reaction solvent.

4. A therapeutic composition active as a solubiliser for bile calculi and as an inhibitor for their formation, comprising the compound of claim 1 as active principle.

**Patentansprüche**

1. Ein Doppelmagnesiumsalz von Chenodesoxycholsäure und Ursodesoxycholsäure der Formel

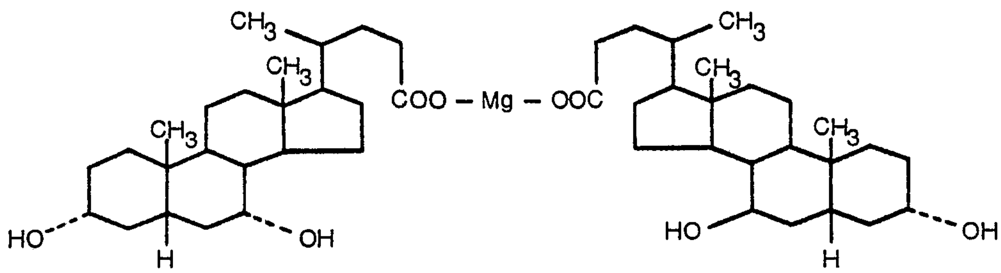

2. Verfahren zum Herstellen der Verbindung wie in Anspruch 1 beansprucht, gekennzeichnet durch Umsetzen eines Magnesium-alkoxids mit Chenodesoxycholsäure in einer wasserfreien alkoholischen Lösung bei einer 20°C nicht übersteigenden Temperatur unter Bildung des Alkoxymagnesiumchenodesoxycholats und anschließendes Umsetzen dieses letzteren in der gleichen alkoholischen Lösung mit Ursodesoxycholsäure.

3. Verfahren, wie in Anspruch 2 beansprucht, unter Verwendung von Magnesiumäthoxid und Äthylalkohol als das Reaktionslösungsmittel.

4. Therapeutische Zusammensetzung, die als Löslichmacher für Gallensteine und als ein Inhibitor für deren Bildung wirksam ist, welche die Verbindung von Anspruch 1 als aktiven Bestandteil enthalt.

**Revendications**

1. Sel magnésien double d'acide chénodésoxycholique et d'acide ursodésoxycholique de formule

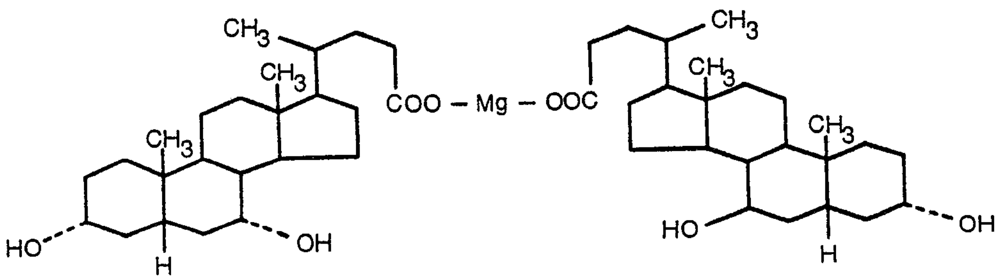

2. Procédé pour la préparation du composé selon la revendication 1, caractérisé en ce qu'on fait réagir un alcoolate de magnésium avec l'acide chénodésoxycholique dans une solution alcoolique anhydre à une

température ne dépassant pas 20°C, pour former le chénodésoxycholate d'alcoxy-magnésium, puis on fait réagir ce dernier, dans la même solution alcoolique, avec l'acide ursodésoxycholique.

3. Procédé selon la revendication 2, utilisant l'éthylate de magnésium et l'alcool éthylique en tant que solvant de la réaction.

4. Composition thérapeutique, active en tant que dissolvant des calculs biliaires et en tant qu'inhibiteur de la formation de ceux-ci, comprenant le composé de la revendication 1 comme principe actif.